(19) **Europäisches Patentamt / European Patent Office / Office européen des brevets**

(11) **EP 3 858 785 A1**

(12) # EUROPEAN PATENT APPLICATION
published in accordance with Art. 153(4) EPC

(43) Date of publication:
**04.08.2021 Bulletin 2021/31**

(21) Application number: **19864567.3**

(22) Date of filing: **24.09.2019**

(51) Int Cl.:
*C01B 11/02* (2006.01)　　*A61L 9/01* (2006.01)

(86) International application number:
**PCT/JP2019/037311**

(87) International publication number:
**WO 2020/067033 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.09.2018  JP 2018184631**

(71) Applicant: **Amatera, Inc.**
**Aichi-gun, Aichi 470-0161 (JP)**

(72) Inventors:
• **FUJITA, Hiromasa**
  **Aichi-gun, Aichi 470-0161 (JP)**
• **FUJITA, Tetsuhiro**
  **Aichi-gun, Aichi 470-0161 (JP)**
• **FUJITA, Masashi**
  **Aichi-gun, Aichi 470-0161 (JP)**
• **TAKATOMI, Hiroshi**
  **Aichi-gun, Aichi 470-0161 (JP)**

(74) Representative: **Vossius & Partner**
**Patentanwälte Rechtsanwälte mbB**
**Siebertstraße 3**
**81675 München (DE)**

(54) **AQUEOUS CHLORINE DIOXIDE SOLUTION AND METHOD FOR PRODUCING SAME**

(57)　The aqueous chlorine dioxide solution comprises chlorine dioxide, a potassium salt of a weak acid having a pKa of 2.5 or more, and a chlorite, and has a pH of 4.0 or more and 7.5 or less. The method for producing the aqueous chlorine dioxide solution comprises a first step of providing a first aqueous solution containing chlorine dioxide, a second step of mixing the first aqueous solution and a second aqueous solution containing a potassium salt of a weak acid having a pKa of 2.5 or more to prepare an intermediate aqueous solution having a pH of 4.0 or more and 7.5 or less, and a third step of mixing the intermediate aqueous solution and a third aqueous solution containing a chlorite, or mixing the intermediate aqueous solution and the third aqueous solution and then further adding the second aqueous solution thereto, to prepare an aqueous chlorine dioxide solution having a pH of 4.0 or more and 7.5 or less. Therefore, an aqueous chlorine dioxide solution in which the concentration of chlorine dioxide is maintained stably without using phosphoric acid or a salt thereof as a pH adjuster, and a method for producing the same are provided.

FIG.1

**Description**

TECHNICAL FIELD

The present disclosure relates to an aqueous chlorine dioxide solution, which is widely used for treatment of an allergy-inducing substance such as a pollen, dust, skin flake and fungi, treatment of a harmful substance such as a pathogenic bacteria, virus and a harmful chemical substance (for example, tobacco smoke, and formaldehyde), environmental purification, deodorization of indoor/outdoor and a food product, fungusproofing and antisepsis etc., and relates to a method for producing the same.

BACKGROUND ART

Since chlorine dioxide ($ClO_2$) has strong oxidizing power, it is widely used for treatment of an allergy-inducing substance such as a pollen, dust, skin flake and fungi, treatment of a harmful substance such as a pathogenic bacteria, virus and a harmful chemical substance (for example, tobacco smoke, and formaldehyde), environmental purification, deodorization of indoor/outdoor and a food product, mildewproofing and antisepsis etc. A composition containing useful chlorine dioxide which is used for such a wide range of applications and a method for producing the same have been proposed.

For example, Japanese Patent Laying-Open No. 11-278808 (PTL 1) proposes a pure chlorine dioxide solution having dissolved chlorine dioxide gas, a chlorite and a pH adjuster as constituents, a gel composition containing the above pure chlorine dioxide solution and a highly water-absorbing resin, a foamable composition containing the above pure chlorine dioxide solution and a foaming agent, and a container for containing the above pure chlorine dioxide solution, the above gel composition, and the above foamable composition.

WO 2008/111358 (PTL 2) proposes a stabilized composition for chlorine dioxide capable of maintaining the concentration of chlorine dioxide in an agent substantially constant even when chlorine dioxide is continuously released as gas from the agent containing dissolved chlorine dioxide, wherein the composition comprises a combination of a chlorite and a pH adjuster, and the pH adjuster is an acid having buffering properties by which the pH of 5% aqueous solution at 25°C is adjusted to 2.5 to 6.8, or a salt thereof.

CITATION LIST

PATENT LITERATURE

PTL 1: Japanese Patent Laying-Open No. 11-278808
PTL 2: WO 2008/111358

SUMMARY OF INVENTION

TECHNICAL PROBLEM

According to Japanese Patent Laying-Open No. 11-278808 (PTL 1), since citric acid is used as a pH adjuster, there is a problem in that the initial concentration of chlorine dioxide in the pure chlorine dioxide solution, the gel composition containing the above pure chlorine dioxide solution and a highly water-absorbing resin, and the foamable composition containing the above pure chlorine dioxide solution and a foaming agent is higher, and the subsequent concentration of chlorine dioxide decreases more.

According to WO 2008/111358 (PTL 2), since phosphoric acid or a salt thereof is used as a pH adjuster, there is a problem of concern about the load imposed on the environment, though the concentration of chlorine dioxide in the stabilized composition for chlorine dioxide can be substantially maintained constant in the wide pH range of pH 2.5 to 6.8.

For the purpose of solving the above problems, an object of the present disclosure is to provide an aqueous chlorine dioxide solution in which the concentration of chlorine dioxide is maintained stably without using phosphoric acid or a salt thereof as a pH adjuster, and a method for producing the same.

SOLUTION TO PROBLEM

The aqueous chlorine dioxide solution according to one embodiment of the present disclosure contains chlorine dioxide, a potassium salt of a weak acid having a pKa of 2.5 or more, and a chlorite, and has a pH of 4.0 or more and 7.5 or less.

In the aqueous chlorine dioxide solution, the potassium salt of the weak acid may be potassium citrate.

In the aqueous chlorine dioxide solution, the concentration of potassium may be 10 ppm or more.

The method for producing the aqueous chlorine dioxide solution according to one embodiment of the present disclosure comprises a first step of providing a first aqueous solution containing chlorine dioxide, a second step of mixing the first aqueous solution and a second aqueous solution containing a potassium salt of a weak acid having a pKa of 2.5 or more to prepare an intermediate aqueous solution having a pH of 4.0 or more and 7.5 or less, and a third step of mixing the intermediate aqueous solution and a third aqueous solution containing a chlorite, or mixing the intermediate aqueous solution and the third aqueous solution and then further adding the second aqueous solution thereto, to prepare an aqueous chlorine dioxide solution having a pH of 4.0 or more and 7.5 or less.

In the method for producing the aqueous chlorine dioxide solution, the potassium salt of the weak acid may be potassium citrate.

ADVANTAGEOUS EFFECT OF INVENTION

According to the present disclosure, it is possible to provide an aqueous chlorine dioxide solution in which the concentration of chlorine dioxide is maintained stably without using phosphoric acid or a salt thereof as a pH adjuster, and a method for producing the same.

BRIEF DESCRIPTION OF DRAWINGS

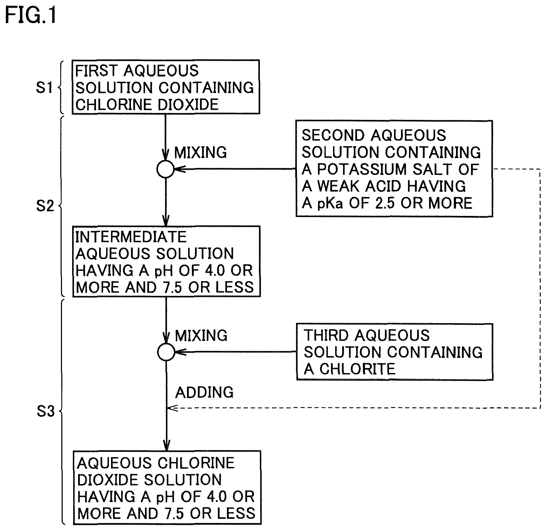

Fig. 1 is a flow chart showing one example of a method for producing the aqueous chlorine dioxide solution according to one embodiment of the present disclosure.

DESCRIPTION OF EMBODIMENTS

<Embodiment 1: Aqueous chlorine dioxide solution>

The aqueous chlorine dioxide solution of an embodiment of the present disclosure contains chlorine dioxide, a potassium salt of a weak acid having a pKa of 2.5 or more, and a chlorite, and has a pH of 4.0 or more and 7.5 or less. Since the aqueous chlorine dioxide solution of the present embodiment contains chlorine dioxide, a potassium salt of a weak acid having a pKa of 2.5 or more as a pH adjuster, and a chlorite, and has pH adjusted to 4.0 or more and 7.5 or less, the concentration of chlorine dioxide is stably maintained without using phosphoric acid and a salt thereof as a pH adjuster. In this case, since the pKa of phosphoric acid is 2.1, the aqueous chlorine dioxide solution of the Embodiment 1 does not contain any of phosphoric acid and a salt thereof.

(Chlorine dioxide)

Chlorine dioxide ($ClO_2$) contained in the aqueous chlorine dioxide solution exists as dissolved chlorine dioxide which is dissolved in the aqueous chlorine dioxide solution. In the atmospheric air at room temperature (for example 25°C), dissolved chlorine dioxide in the aqueous chlorine dioxide solution decreases by being partially released as gas outside the aqueous solution (into the atmospheric air), but is supplemented by chlorite ion ($ClO_2^-$) in a chlorite due to pH equilibrium as described below.

The aqueous chlorine dioxide solution exhibits effects in treatment of an allergy-inducing substance such as a pollen, dust, skin flake and fungi, treatment of a harmful substance such as a pathogenic bacteria, virus and a harmful chemical substance (for example, tobacco smoke, and formaldehyde), environmental purification, deodorization of indoor/outdoor and a food product, mildewproofing and antisepsis etc., which are resulted from the above dissolved chlorine dioxide and the above chlorine dioxide released as a gas outside the aqueous solution.

The concentration of chlorine dioxide contained in the aqueous chlorine dioxide solution is not particularly limited, but is preferably 10 ppm or more, more preferably 50 ppm or more, further preferably 100 ppm or more in view of enhancing the above effects resulted from chlorine dioxide, and is preferably 2000 ppm or less, more preferably 1000 ppm or less, further preferably 400 ppm or less in view of stably maintaining the concentration of chlorine dioxide. The concentration of chlorine dioxide ($ClO_2$) contained in the aqueous chlorine dioxide solution is obtained by adding 0.5 g of potassium iodide and 1 ml of buffer aqueous solution of pH 7 to 190 to 200 ml of ion exchanged water, then adding 1 to 10 ml of aqueous chlorine dioxide solution thereto, immediately titrating the obtained solution with 0.1 N aqueous solution of sodium thiosulfate, and calculating the concentration from the titer A ml using the following formula (1).

$$(\text{ClO}_2\ [\text{ppm}]) = A \times 0.1 \times 67450/(\text{mass of aqueous chlorine dioxide solution}$$
$$[g]) \dots (1)$$

As the above buffer aqueous solution of pH 7, the solution obtained by dissolving 4.57 g of disodium hydrogen phosphate ($Na_2HPO_4$) and 9.228 g of potassium dihydrogen phosphate ($KH_2PO_4$) in ion exchanged water so as to provide the total amount of 1000 ml is used.

(Potassium salt of a weak acid having a pKa of 2.5 or more)

The potassium salt (K salt) of a weak acid having a pKa of 2.5 or more contained in the aqueous chlorine dioxide solution is preferably a potassium salt of a polybasic acid, for example potassium citrate which is a potassium salt of citric acid ($pKa_1$ is 2.90, $pKa_2$ is 4.35, $pKa_3$ is 5.69), potassium malate which is a potassium salt of malic acid ($pKa_1$ is 3.23, $pKa_2$ is 4.77), potassium succinate which is a potassium salt of succinic acid ($pKa_1$ is 3.99, $pKa_2$ is 5.20), more preferably potassium citrate from the viewpoint that the potassium salt stably maintains the concentration of chlorine dioxide contained in the aqueous chlorine dioxide solution as a pH adjuster. As potassium citrate, 3 types of potassium citrate, i.e. potassium citrate monobasic (monopotassium citrate), potassium citrate dibasic (dipotassium citrate) and tripotassium citrate are included, and among these, potassium citrate dibasic and tripotassium citrate are more preferable. A potassium salt may be an anhydride salt or may be a salt hydrate. In the case of a polybasic acid, "pKa of 2.5 or more" means that every $pKa_k$ (k is an integer of 1 or more) at each step is 2.5 or more, that is, the minimum $pKa_k$ is 2.5 or more.

As a pH adjuster, a sodium salt (Na salt) of a weak acid is generally used since it is easily available. At first, the inventors produced the aqueous chlorine dioxide solution containing chlorine dioxide, a chlorite and sodium citrate which is a sodium salt of a weak acid having a pKa of 2.5 or more as a pH adjuster, but the concentration of chlorine dioxide in the aqueous chlorine dioxide solution is difficult to maintain stably.

The details of the reason why the concentration of chlorine dioxide in the aqueous chlorine dioxide solution can be stably maintained when a potassium salt of a weak acid having a pKa of 2.5 or more (K salt) is used as a pH adjuster instead of a sodium salt (Na salt) of a weak acid having a pKa of 2.5 or more is unknown. However, while a sodium salt forms a bicarbonate salt in an aqueous solution containing carbonic acid generated by dissolution of carbon dioxide in the atmospheric air, a potassium salt does not form a bicarbonate salt, and potassium is more wettable with water and well hydrated to exist more stably in an aqueous solution than sodium, and thus it is considered that the pH of the aqueous chlorine dioxide solution can be maintained more stably within the specified range (specifically, within the pH range of 4.0 or more and 7.5 or less) due to these factors. The present inventors completed the present invention by discovering the remarkable effect in which the concentration of chlorine dioxide in the aqueous chlorine dioxide solution can be maintained very stably by using a potassium salt of a weak acid having a pKa of 2.5 or more as a pH adjuster instead of a sodium salt of a weak acid having a pKa of 2.5 or more, and which had not been predicted even by those skilled in the art.

The potassium salt of a weak acid having a pKa of 2.5 or more contained in the aqueous chlorine dioxide solution dissociates in the aqueous solution to generate the weak acid ion having a pKa of 2.5 or more and potassium (K) ion. The concentration of potassium existing as an ion in the aqueous chlorine dioxide solution is not particularly limited, but preferably 10 ppm or more, more preferably 100 ppm or more, further preferably 200 ppm or more, particularly preferably 500 ppm or more from the view point that the concentration of chlorine dioxide is maintained stably by pH buffering effect, and preferably 10% by mass (100000 ppm) or less, more preferably 1% by mass (10000 ppm) or less, further preferably 5000 ppm or less, particularly preferably 2500 ppm or less in view of suppressing the enhancement of pH buffering effect. The concentration of potassium contained in the aqueous chlorine dioxide solution is measured by fluorescence X-ray analysis, atomic absorption spectroscopy etc.

(Chlorite)

The chlorite contained in the aqueous chlorine dioxide solution is not particularly limited as long as it can dissociate to generate chlorite ion ($ClO_2^-$) which can supplement chlorine dioxide due to pH equilibrium in the aqueous solution, and examples of the chlorite include chlorites of group 1 elements except for hydrogen (alkali metal elements) such as sodium chlorite ($NaClO_2$), potassium chlorite ($KClO_2$) and lithium chlorite ($LiClO_2$), and chlorites of group 2 elements such as calcium chlorite ($Ca(ClO_2)_2$), strontium chlorite ($Sr(ClO_2)_2$), barium chlorite ($Ba(ClO_2)_2$) and magnesium chlorite ($Mg(ClO_2)_2$). Among these, chlorites of group 1 elements except for hydrogen (alkali metal elements) are preferable because of high dissociation degree, and furthermore, potassium chlorite is more preferable since it is easily hydrated, but among chlorites of group 1 elements except for hydrogen (alkali metal elements), sodium chlorite is particularly easily available and does not have any problem when being used.

The chlorite contained in the aqueous chlorine dioxide solution dissociates in the aqueous solution to generate chlorite ion ($ClO_2^-$) and a counter ion. The concentration of such chlorite ion is not particularly limited, but preferably 100 ppm or more, more preferably 500 ppm or more, further preferably 2000 ppm or more from the viewpoint that the concentration of chlorine dioxide is maintained stably by supplementing chlorine dioxide from chlorite ion by pH equilibrium, and preferably 10% by mass (100000 ppm) or less, more preferably 2.5% by mass (25000 ppm) or less, further preferably 1% by mass (10000 ppm) or less in view of suppressing the generation of excess chlorite. The concentration of chlorite ion ($ClO_2^-$) contained in the aqueous chlorine dioxide solution is obtained by adding 2 to 3 ml of 2.5 N aqueous solution of hydrochloric acid to the aqueous solution used in titration for measurement of the concentration of chlorine dioxide ($ClO_2$) contained in the above aqueous chlorine dioxide solution, leaving the obtained solution to stand in a dark place for 5 minutes, then titrating the solution with 0.1 N aqueous solution of sodium thiosulfate again, and calculating the concentration from the titer B ml using the following formula (2).

$$(ClO_2^- \, [ppm]) = (B/4\text{-}A) \times 0.1 \times 67450/(\text{mass of aqueous chlorine dioxide solution } [g]) \ldots (2)$$

A in formula (2) is same as A in formula (1).

(pH of aqueous chlorine dioxide solution)

The pH of the aqueous chlorine dioxide solution is 4.0 or more and 7.5 or less due to pH buffering effect of the potassium salt having a pKa of 2.5 or more as a pH adjuster. The pH of the aqueous chlorine dioxide solution is 4.0 or more, preferably 5.0 or more, more preferably 5.5 or more, from the viewpoint that the concentration of chlorine dioxide is maintained stably, and is 7.5 or less, preferably less than 7.0, more preferably 6.5 or less in view of enhancing the above effect resulted from chlorine dioxide.

<Embodiment 2: method for producing aqueous chlorine dioxide solution>

In reference to Fig. 1, the method for producing the aqueous chlorine dioxide solution which is another embodiment of the present disclosure comprises a first step S1 of providing a first aqueous solution containing chlorine dioxide, a second step S2 of mixing the first aqueous solution and a second aqueous solution containing a potassium salt of a weak acid having a pKa of 2.5 or more to prepare an intermediate aqueous solution having a pH of 4.0 or more and 7.5 or less, and a third step S3 of mixing the intermediate aqueous solution and a third aqueous solution containing a chlorite, or mixing the intermediate aqueous solution and the third aqueous solution and then further adding the second aqueous solution thereto, to prepare the aqueous chlorine dioxide solution having a pH of 4.0 or more and 7.5 or less. In the method for producing the aqueous chlorine dioxide solution of the present embodiment, stabilized chlorine dioxide having a pH of 4.0 or more and 7.5 or less in which the concentration of chlorine dioxide is maintained stably can be efficiently produced by sequentially mixing the first aqueous solution containing chlorine dioxide, the second aqueous solution containing the first aqueous solution and a potassium salt of a weak acid having a pKa of 2.5 or more, and the third aqueous solution containing a chlorite while maintaining the pH within the specified range (specifically within the pH range of 4.0 or more and 7.5 or less).

(First step)

In the first step S1, the first aqueous solution containing chlorine dioxide is provided. The first aqueous solution containing chlorine dioxide can be produced by a conventionally known method for producing an aqueous solution containing dissolved chlorine dioxide. For example, an aqueous solution containing chlorine dioxide (specifically dissolved chlorine dioxide) can be prepared by mixing an aqueous solution containing a chlorite (for example sodium chlorite) and an aqueous solution containing an acid (for example hydrochloric acid) by a conventionally known method so as to react the chlorite with the acid.

The concentration of chlorine dioxide contained in the first aqueous solution is not particularly limited, but is preferably 500 ppm or more, more preferably 1000 ppm or more, further preferably 2000 ppm or more from the viewpoint that the concentration of chlorine dioxide is maintained stably, and is preferably 5% by mass (50000 ppm) or less, more preferably 2% by mass (20000 ppm) or less, further preferably 1% by mass (10000 ppm) or less from the viewpoint that the concentration of chlorine dioxide is maintained stably. The concentration of chlorine dioxide contained in the first aqueous solution is measured by the similar method to the above method for measurement of the concentration of chlorine dioxide contained in the aqueous chlorine dioxide solution. The pH of the first aqueous solution is preferably 4.0 or more and 7.5 or less in view of facilitating pH adjustment in the producing steps. A method of pH measurement of the first to the third aqueous solution and the intermediate aqueous solution is not particularly limited, and a conventionally known method such as a method using a pH meter can be adopted.

(Second step)

In the second step S2, the first aqueous solution and the second aqueous solution containing a potassium salt of a weak acid having a pKa of 2.5 or more are mixed to prepare the intermediate aqueous solution having a pH of 4.0 or more and 7.5 or less. A method of mixing the first aqueous solution and the second aqueous solution is not particularly limited, and a conventionally known mixing method can be adopted.

The potassium salt of a weak acid having a pKa of 2.5 or more contained in the second aqueous solution dissociates in the aqueous solution to generate the weak acid ion having a pKa of 2.5 or more and potassium (K) ion. The concentration of potassium existing as an ion in the second aqueous solution is not particularly limited, but is preferably 0.1% by mass (1000 ppm) or more, more preferably 1% by mass (10000 ppm), further preferably 2% by mass (20000 ppm) or more from the viewpoint that pH buffering effect is maintained at a high level, and is preferably 30% by mass (300000 ppm) or less, more preferably 20% by mass (200000 ppm) or less, further preferably 10% by mass (100000 ppm) or less in view of suppressing the enhancement of pH buffering effect. The concentration of potassium contained in the second aqueous solution is measured by fluorescence X-ray analysis, atomic absorption spectroscopy etc. The pH of the second aqueous solution is preferably 4.0 or more and 7.5 or less in view of facilitating pH adjustment in the producing steps. As described in Embodiment 1, a potassium salt of a weak acid having a pKa of 2.5 or more is preferably potassium citrate, potassium malate, potassium succinate, etc., more preferably potassium citrate. As such potassium citrate, 3 types of potassium citrate, i.e. potassium citrate monobasic (monopotassium citrate), potassium citrate dibasic (dipotassium citrate) and tripotassium citrate are included, and among these, potassium citrate dibasic and tripotassium citrate are more preferable.

(Third step)

In the third step S3, the intermediate aqueous solution and the third aqueous solution containing a chlorite are mixed, or the intermediate aqueous solution and the third aqueous solution are mixed and then further the second aqueous solution is added thereto, to prepare the aqueous chlorine dioxide solution having a pH of 4.0 or more and 7.5 or less. A method of mixing the intermediate aqueous solution and the third aqueous solution is not particularly limited, and a known mixing method can be adopted. A method of further adding the second aqueous solution to the above mixed solution is not particularly limited, and a known adding method can be adopted.

The chlorite contained in the third aqueous solution dissociates in the aqueous solution to generate chlorite ion ($ClO_2^-$) and a counter ion. The concentration of such chlorite ion is not particularly limited, but preferably 250 ppm or more, more preferably 1000 ppm or more, further preferably 3000 ppm or more from the viewpoint that the concentration of chlorine dioxide is maintained stably by supplementing chlorine dioxide from chlorite ion by pH equilibrium, and preferably 20% by mass (200000 ppm) or less, more preferably 15% by mass (150000 ppm) or less, further preferably 10% by mass (100000 ppm) or less in view of suppressing generation of excess chlorite. The concentration of chlorite ion contained in the third aqueous solution is measured by the similar method to the above method for measurement of the concentration of chlorite ion contained in the aqueous chlorine dioxide solution. The pH of the third aqueous solution is preferably 4.0 or more and 7.5 or less in view of facilitating pH adjustment in the producing steps.

In the method for producing the aqueous chlorine dioxide solution of the present embodiment, the pH of the intermediate aqueous solution is 4.0 or more and 7.5 or less from the viewpoint that the aqueous chlorine dioxide solution having a pH of 4.0 or more and 7.5 or less is prepared stably and efficiently. For further details, the pH of the intermediate aqueous solution is 4.0 or more, preferably 5.0 or more, more preferably 5.5 or more in view of suppressing an increase in the concentration of chlorine dioxide to stably maintain the concentration of chlorine dioxide also during production. Furthermore, the pH of the intermediate aqueous solution is 7.5 or less, preferably less than 7.0, more preferably 6.5 or less in view of suppressing a decrease in the concentration of chlorine dioxide to stably maintain the concentration of chlorine dioxide also during production.

EXAMPLES

(Example 1)

1. Production of aqueous chlorine dioxide solution

In reference to Fig. 1, at first, in the first step S1, ion exchanged water was added to the aqueous chlorine dioxide solution obtained by reacting 16% by mass aqueous solution of sodium chlorite (80 ml/min) with 10% by mass aqueous solution of hydrochloric acid (77 ml/min) under a pressurizing condition (1 MPa) to thereby prepare a high concentration aqueous chlorine dioxide solution containing 4995 ppm of chlorine dioxide. 5092 g of the high concentration aqueous chlorine dioxide solution was diluted by being gently flowed into 90735 g of ion exchanged water to prepare 95827 g of the first aqueous solution containing 265 ppm of chlorine dioxide. Then, in the second step S2, the above first aqueous solution and 2405 g of second aqueous solution containing 12.6% by mass (126000 ppm) of tripotassium citrate as a pH adjuster were gently mixed by propeller stirring to prepare 98232 g of intermediate aqueous solution having a pH of 6.10. In this case, the second aqueous solution was obtained by uniformly dissolving 432 g of tripotassium citrate monohydrate in 3000 g of ion exchanged water. Then, in the third step S3, the above intermediate aqueous solution and 2910 g of third aqueous solution containing 25% by mass (250000 ppm) of sodium chlorite were gently mixed by propeller stirring to produce 101142 g of aqueous chlorine dioxide solution having a pH of 6.06. When the pH of the aqueous chlorine dioxide solution was not within the specified range (specifically within the pH range of 4.0 or more and 7.5 or less, preferably 5.0 or more and less than 7.0, more preferably 5.5 or more and 6.5 or less) after mixing the above intermediate aqueous solution and the above third aqueous solution, the above second aqueous solution was further added in an appropriate amount to the mixed solution to adjust the pH of the aqueous chlorine dioxide solution to the specified range (specifically within the pH range of 4.0 or more and 7.5 or less, preferably 5.0 or more and less than 7.0, more preferably 5.5 or more and 6.5 or less).

2. Changes over time in concentrations of chlorine dioxide and chlorite ion and pH in aqueous chlorine dioxide solution

300 g of aqueous chlorine dioxide solution obtained above was put into a 300 ml propylene container equipped with a screw cap, and the container was sealed by applying 2.5 N of torque pressure to the screw cap. Changes over time in the concentrations of chlorine dioxide ($ClO_2$) and chlorite ion ($ClO_2^-$) in the aqueous chlorine dioxide solution in the container were investigated for the respective case of leaving the sealed container in a thermostatic chamber at 20°C or a thermostatic chamber at 50°C. The concentration of chlorine dioxide in the aqueous chlorine dioxide solution was measured as follows.

The concentration of chlorine dioxide ($ClO_2$) contained in the aqueous chlorine dioxide solution is obtained by adding 0.5 g of potassium iodide and 1 ml of buffer aqueous solution of pH 7 to 190 to 200 ml of ion exchanged water, then adding 1 to 10 ml of aqueous chlorine dioxide solution thereto, immediately titrating the obtained solution with 0.1 N aqueous solution of sodium thiosulfate, and calculating the concentration from the titer A ml using the following formula (1).

$$(ClO_2 \text{ [ppm]}) = A \times 0.1 \times 67450/(\text{mass of aqueous chlorine dioxide solution [g]}) \dots (1)$$

As the above buffer aqueous solution of pH 7, the solution obtained by dissolving 4.57 g of disodium hydrogen phosphate ($Na_2HPO_4$) and 9.228 g of potassium dihydrogen phosphate ($KH_2PO_4$) in ion exchanged water so as to provide the total amount of 1000 ml is used.

The concentration of chlorite ion ($ClO_2^-$) contained in the aqueous chlorine dioxide solution was obtained by adding 2 to 3 ml of 2.5 N aqueous solution of hydrochloric acid to the aqueous solution used in titration for measurement of the concentration of chlorine dioxide ($ClO_2$) contained in the above aqueous chlorine dioxide solution, leaving the obtained solution to stand in a dark place for 5 minutes, then titrating the solution with 0.1 N aqueous solution of sodium thiosulfate again, and calculating the concentration from the titer B ml using the following formula (2).

$$(ClO_2^- \text{ [ppm]}) = (B/4-A) \times 0.1 \times 67450/(\text{mass of aqueous chlorine dioxide solution [g]}) \dots (2)$$

A in formula (2) is same as A in formula (1).

In order to calculate the mass of aqueous chlorine dioxide solution used for titration in the above formulae (1) and (2), the specific gravity of the aqueous chlorine dioxide solution was measured as follows. That is, 100 ml of the aqueous chlorine dioxide solution was measured out by a measuring cylinder, the weight thereof was measured, and the obtained weight was divided by the measured volume to calculate the specific gravity. The calculated specific gravity was 1.02. The pH of the aqueous chlorine dioxide solution was measured by a pH meter (PH/ORP METER D-72 manufactured by HORIBA, Ltd.). The concentration of potassium contained in the initial aqueous chlorine dioxide solution was 1100 ppm when it was measured by fluorescence X-ray analysis and atomic absorption spectroscopy, and the concentration was well coincided with the calculated value 1082 ppm, which was the concentration of potassium in the aqueous chlorine dioxide solution calculated from the concentration of potassium in the second aqueous solution.

Changes over time in the concentrations of chlorine dioxide ($ClO_2$) and chlorite ion ($ClO_2^-$) contained in the aqueous chlorine dioxide solution in the above container and pH of the aqueous chlorine dioxide solution were summarized for the respective case of leaving the container in a thermostatic chamber at 20°C or a thermostatic chamber at 50°C.

[Table 1]

| Condition of still-standing | Measurement item | Initial stage | 7 days later | Amount of change |
|---|---|---|---|---|
| 20°C | $ClO_2$ concentration (ppm) | 214 | 211 | -3 |
| | $ClO_2^-$ concentration (ppm) | 5081 | 5123 | +42 |
| | pH | 6.06 | 6.08 | +0.02 |
| 50°C | $ClO_2$ concentration (ppm) | 214 | 214 | 0 |
| | $ClO_2^-$ concentration (ppm) | 5081 | 4991 | -90 |
| | pH | 6.06 | 6.48 | +0.42 |

In reference to Table 1, in the aqueous chlorine dioxide solution containing chlorine dioxide, a potassium salt of a weak acid having a pKa of 2.5 or more (for example potassium citrate) as a pH adjuster and a chlorite and having a pH of 4.0 or more and 7.5 or less, change over time in the concentration of chlorine dioxide ($ClO_2$) was small under any condition of still-standing at atmosphere temperature of 20°C and 50°C, and the concentration of chlorine dioxide was maintained stably. Furthermore, decrease in pH was not observed in any condition of still-standing at atmosphere temperature of 20°C and 50°C. In the condition of still-standing at atmosphere temperature at 50°C, the concentration of chlorite ion ($ClO_2^-$) decreased, and it was interpreted that chlorine dioxide was supplemented by chlorite ion in the chlorite.

(Comparative Example 1)

1. Production of aqueous chlorine dioxide solution

Similarly to Example 1, 5092 g of high concentration aqueous chlorine dioxide solution containing 4995 ppm of chlorine dioxide was prepared, which was then diluted by being gently flowed into 90735 g of ion exchanged water to prepare 95827 g of the aqueous chlorine dioxide solution containing 195 ppm of chlorine dioxide and having a pH of 6.03.

2. Changes over time in concentrations of chlorine dioxide and chlorite ion and pH in aqueous chlorine dioxide solution

Similarly to Example 1, changes over time in the concentrations of chlorine dioxide ($ClO_2$) and chlorite ion ($ClO_2^-$) contained in the aqueous chlorine dioxide solution and the pH of the aqueous chlorine dioxide solution were measured for the aqueous chlorine dioxide solution obtained above, and summarized in Table 2.

[Table 2]

| Condition of still-standing | Measurement item | Initial stage | 7 days later | Amount of change |
|---|---|---|---|---|
| 20°C | $ClO_2$ concentration (ppm) | 195 | 139 | -56 |
| | $ClO_2^-$ concentration (ppm) | 348 | 348 | 0 |
| | pH | 6.03 | 5.90 | -0.13 |
| 50°C | $ClO_2$ concentration (ppm) | 195 | 120 | -75 |
| | $ClO_2^-$ concentration (ppm) | 348 | 348 | 0 |
| | pH | 6.03 | 5.87 | -0.16 |

In reference to Table 2, in the aqueous chlorine dioxide solution not containing any pH adjuster, the concentration of chlorine dioxide ($ClO_2$) significantly decreased and decrease in pH was observed under any condition of still-standing at atmosphere temperature of 20°C and 50°C. Change over time in the concentration of chlorite ion generated from dissociation of chlorine dioxide was not observed.

(Comparative Example 2)

1. Production of aqueous chlorine dioxide solution

At first, in the first step, ion exchanged water was added to the aqueous chlorine dioxide solution obtained by reacting 16% by mass aqueous solution of sodium chlorite (80 ml/min) with 10% by mass aqueous solution of hydrochloric acid (77 ml/min) under a pressurizing condition (1 MPa) to thereby prepare a high concentration aqueous chlorine dioxide solution containing 4995 ppm of chlorine dioxide. 432 g of the high concentration aqueous chlorine dioxide solution was diluted by being gently flowed into 9040 g of ion exchanged water to prepare 9472 g of the first aqueous solution containing 228 ppm of chlorine dioxide. Then, in the second step, the above first aqueous solution and 261 g of second aqueous solution containing 12.6% by mass (126000 ppm) of tripotassium citrate as a pH adjuster were gently mixed by propeller stirring to prepare 9733 g of intermediate aqueous solution having a pH of 6.10. In this case, the second aqueous solution was obtained by uniformly dissolving 108 g of trisodium citrate dihydrate in 750 g of ion exchanged water. Then, in the third step, the above intermediate aqueous solution and 288 g of third aqueous solution containing 25% by mass (250000 ppm) of sodium chlorite were gently mixed by propeller stirring to produce 10021 g of aqueous chlorine dioxide solution having a pH of 6.10. When the pH of the aqueous chlorine dioxide solution was not within the specified range (specifically within the pH range of 4.0 or more and 7.5 or less, preferably 5.0 or more and less than 7.0, more preferably 5.5 or more and 6.5 or less) after mixing the above intermediate aqueous solution and the above third aqueous solution, the above second aqueous solution was further added in an appropriate amount to the mixed solution to adjust the pH of the aqueous chlorine dioxide solution to the specified range (specifically within the pH range of 4.0 or more and 7.5 or less, preferably 5.0 or more and less than 7.0, more preferably 5.5 or more and 6.5 or less).

2. Changes over time in concentrations of chlorine dioxide and chlorite ion and pH in aqueous chlorine dioxide solution

Similarly to Example 1, changes over time in the concentrations of chlorine dioxide ($ClO_2$) and chlorite ion ($ClO_2^-$) contained in the aqueous chlorine dioxide solution and pH of the aqueous chlorine dioxide solution were measured for the aqueous chlorine dioxide solution obtained above, and summarized in Table 3.

[Table 3]

| Condition of still-standing | Measurement item | Initial stage | 7 days later | Amount of change |
|---|---|---|---|---|
| 20°C | $ClO_2$ concentration (ppm) | 195 | 182 | -13 |
| | $ClO_2^-$ concentration (ppm) | 5108 | 5095 | -13 |
| | pH | 6.10 | 6.13 | +0.03 |
| 50°C | $ClO_2$ concentration (ppm) | 195 | 220 | +25 |
| | $ClO_2^-$ concentration (ppm) | 5108 | 5007 | -101 |
| | pH | 6.10 | 6.52 | +0.42 |

In reference to Table 3, in the aqueous chlorine dioxide solution containing chlorine dioxide, a sodium salt of a weak acid having a pKa of 2.5 or more (for example sodium citrate) as a pH adjuster and a chlorite, and having a pH of 4.0 or more and 7.5 or less, the change over time in the concentration of chlorine dioxide ($ClO_2$) was smaller than that in Comparative Example 1 but larger than Example 1 in any condition of still-standing at atmosphere temperature of 20°C and 50°C, and the concentration was not maintained stably. Decrease in pH was not observed under any condition of still-standing at atmosphere temperature of 20°C and 50°C. In the condition of still-standing at atmosphere temperature of 50°C, the concentration of chlorite ion ($ClO_2^-$) decreased, and thus it was interpreted that chlorine dioxide was supplemented by chlorite ion in the chlorite.
As apparent from comparison between Comparative Example 2 and Example 1, in the aqueous chlorine dioxide solution using a sodium salt of a weak acid having a pKa of 2.5 or more (for example trisodium citrate) as a pH adjuster, the concentration of chlorine dioxide ($ClO_2$) was unstable, but in the aqueous chlorine dioxide solution using a potassium salt of a weak acid having a pKa of 2.5 or more (for example tripotassium citrate) as a pH adjuster, the concentration of chlorine dioxide ($ClO_2$) was maintained stably.
It should be understood that the embodiments and Examples disclosed herein are illustrative and non-restrictive in all respects. The scope of the invention is defined by the claims, rather than the embodiments and Examples above, and is intended to encompass all modifications within the meanings and the scope equivalent to the claims.

REFERENCE SIGNS LIST

S1 First step, S2 Second step, S3 Third step.

**Claims**

1. An aqueous chlorine dioxide solution comprising chlorine dioxide, a potassium salt of a weak acid having a pKa of 2.5 or more, and a chlorite, the aqueous chlorine dioxide solution having a pH of 4.0 or more and 7.5 or less.

2. The aqueous chlorine dioxide solution according to claim 1, wherein the potassium salt of the weak acid is potassium citrate.

3. The aqueous chlorine dioxide solution according to claim 1 or claim 2, wherein a concentration of potassium is 10 ppm or more.

4. A method for producing an aqueous chlorine dioxide solution, comprising
a first step of providing a first aqueous solution containing chlorine dioxide,
a second step of mixing the first aqueous solution and a second aqueous solution containing a potassium salt of a weak acid having a pKa of 2.5 or more to prepare an intermediate aqueous solution having a pH of 4.0 or more and 7.5 or less, and
a third step of mixing the intermediate aqueous solution and a third aqueous solution containing a chlorite, or mixing the intermediate aqueous solution and the third aqueous solution and then further adding the second aqueous solution thereto, to prepare an aqueous chlorine dioxide solution having a pH of 4.0 or more and 7.5 or less.

5. The method for producing the aqueous chlorine dioxide solution according to claim 4, wherein the potassium salt of the weak acid is potassium citrate.

# FIG.1

S1 { FIRST AQUEOUS SOLUTION CONTAINING CHLORINE DIOXIDE

MIXING

SECOND AQUEOUS SOLUTION CONTAINING A POTASSIUM SALT OF A WEAK ACID HAVING A pKa OF 2.5 OR MORE

S2 { INTERMEDIATE AQUEOUS SOLUTION HAVING A pH OF 4.0 OR MORE AND 7.5 OR LESS

MIXING

THIRD AQUEOUS SOLUTION CONTAINING A CHLORITE

ADDING

S3 { AQUEOUS CHLORINE DIOXIDE SOLUTION HAVING A pH OF 4.0 OR MORE AND 7.5 OR LESS

EP 3 858 785 A1

# INTERNATIONAL SEARCH REPORT

| International application No. |
|---|
| PCT/JP2019/037311 |

**A. CLASSIFICATION OF SUBJECT MATTER**
Int.Cl. C01B11/02(2006.01)i, A61L9/01(2006.01)i

According to International Patent Classification (IPC) or to both national classification and IPC

**B. FIELDS SEARCHED**

Minimum documentation searched (classification system followed by classification symbols)
Int.Cl. C01B11/02, 11/08-11/10, A61L9/00-9/22, 12/10, A01N59/00, A01P1/00-23/00

Documentation searched other than minimum documentation to the extent that such documents are included in the fields searched
Published examined utility model applications of Japan    1922–1996
Published unexamined utility model applications of Japan  1971–2019
Registered utility model specifications of Japan          1996–2019
Published registered utility model applications of Japan  1994–2019

Electronic data base consulted during the international search (name of data base and, where practicable, search terms used)
JSTPlus/JST7580/JSTChina(JDreamIII), CAplus/REGISTRY(STN), DWPI(Derwent Innovation)

**C. DOCUMENTS CONSIDERED TO BE RELEVANT**

| Category* | Citation of document, with indication, where appropriate, of the relevant passages | Relevant to claim No. |
|---|---|---|
| X | JP 52-123399 A (MINNESOTA MINING AND MANUFACTURING COMPANY) 17 October 1977, claims, examples 1-3, 5, 12-14 | 1-3 |
| A | & US 4104190 A & GB 1579431 A, claims, examples 1-3, 5, 12-14 & DE 2712574 A1 & FR 2345393 A & ES 457072 A & CA 1117272 A & IT 1086732 B | 4-5 |

☒ Further documents are listed in the continuation of Box C.     ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 10.10.2019 | 29.10.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
|---|
| PCT/JP2019/037311 |

C (Continuation).   DOCUMENTS CONSIDERED TO BE RELEVANT

| Category* | Citation of document, with indication, where appropriate, of the relevant passages | Relevant to claim No. |
|---|---|---|
| X<br>A | JP 2017-110277 A (OSAKA SODA CO., LTD.) 22 June 2017, claims, paragraphs [0010]-[0026], examples 1, 2, fig. 1, tables 1, 2<br>(Family: none) | 1-3<br>4-5 |
| A | JP 2016-088797 A (AMATERA KK) 23 May 2016<br>(Family: none) | 1-5 |
| A | JP 1-319408 A (OIKAWA, Kikuo) 25 December 1989<br>& US 5165910 A & EP 347320 A1 & KR 10-1997-0000893 B | 1-5 |
| A | CN 101530625 A (BAI, D.) 16 September 2009<br>(Family: none) | 1-5 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11278808 A

- WO 2008111358 A